(19) Europäisches Patentamt
European Patent Office
Office européen des brevets

(11) Veröffentlichungsnummer: **0 533 012 A1**

# (12) EUROPÄISCHE PATENTANMELDUNG

(21) Anmeldenummer: **92115271.6**

(51) Int. Cl.[5]: **C07D 239/52**

(22) Anmeldetag: **07.09.92**

(30) Priorität: **19.09.91 DE 4131136**

(43) Veröffentlichungstag der Anmeldung:
**24.03.93 Patentblatt 93/12**

(84) Benannte Vertragsstaaten:
**BE CH DE ES FR GB IT LI NL**

(71) Anmelder: **BAYER AG**
**W-5090 Leverkusen 1 Bayerwerk(DE)**

(72) Erfinder: **Riebel, Hans-Jochem, Dr.**
**In der Beek 92**
**W-5600 Wuppertal 1(DE)**

## (54) Verfahren zur Herstellung von S-Alkyl-Isothioureidoazinen.

(57) Die als Zwischenprodukte zur Herstellung von Herbiziden verwendbaren S-Alkyl-isothioureidoazine der Formel (I)

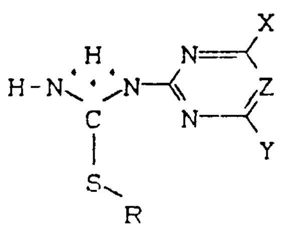

(I)

(worin R für Alkyl und Z für N oder CH steht und die Reste X und Y die in der Beschreibung genannten Bedeutungen haben),
können in guten Ausbeuten hergestellt werden, indem man Thioureidoazine der Formel (II)

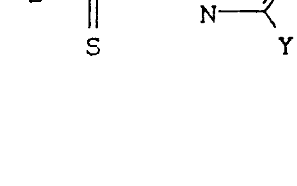

(II)

mit Dialkylsulfaten der Formel

$RO-SO_2-OR$ (III)

gegebenenfalls in Gegenwart eines unpolaren Verdünnungsmittels bei Temperaturen zwischen 20 ° C und 150 ° C umsetzt und die hierbei gebildeten Addukte der Formel (Ia)

EP 0 533 012 A1

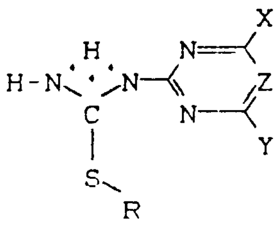

(I)

nach Isolierung oder ohne Zwischenisolierung mit einem Säureakzeptor in Gegenwart eines aprotisch polaren Verdünnungsmittels bei Temperaturen zwischen 0 ° C und 50 ° C umsetzt.

Die Erfindung betrifft ein Verfahren zur Herstellung von bekannten S-Alkyl-isothioureidoazinen, welche als Zwischenprodukte zur Herstellung von Herbiziden verwendet werden können.

Es ist bekannt, daß man S-Alkyl-isothioureidoazine erhält, wenn man Halogenazine mit S-Alkyl-isothiuroniumsalzen umsetzt (vgl. EP-A 117014, Bsp. 9). Die Ausbeute ist bei dieser Herstellungsmethode jedoch unbefriedigend.

Weiter ist bekannt, daß S-Alkyl-isothioureidoazine auch durch Umsetzung von N-Azinyl-iminodithiokohlensäure-diestern mit Ammoniak hergestellt werden können (vgl. EP-A 358018, Bsp. II-1). Die Herstellung der hierbei als Ausgangsstoffe benötigten N-Azinyl-iminodithiokohlensäurediester unter Verwendung von Schwefelkohlenstoff ist jedoch im industriellen Maßstab nur sehr schwierig durchführbar.

Schließlich ist bekannt, daß auch bestimmte Thioureidoazine mit Dimethylsulfat zu entsprechenden S-Methyl-isothioureidoazinen alkyliert werden können (vgl. J. Heterocycl. Chem. 22 (1985), 1147-1148). Das hierbei verwendete Lösungsmittel Dimethylformamid ist jedoch für die technische Anwendung kaum noch akzeptabel.

Es wurde nun gefunden, daß man S-Alkyl-isothioureidoazine der allgemeinen Formel (I)

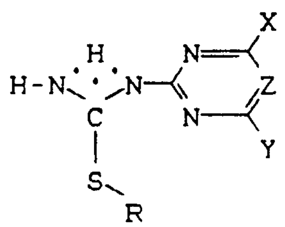

(I)

in welcher

R für Alkyl steht,

X und Y gleich oder verschieden sind und für Wasserstoff, Halogen oder für einen jeweils gegebenenfalls substituierten Rest der Reihe Alkyl, Halogenalkyl, Alkoxyalkyl, Halogenalkoxy, Alkoxy, Alkylthio, Halogenalkylthio, Alkylamino oder Dialkylamino stehen, und

Z für Stickstoff oder eine CH-Gruppierung steht,

in sehr guten Ausbeuten und in hoher Reinheit erhält, wenn man Thioureidoazine der allgemeinen Formel (II)

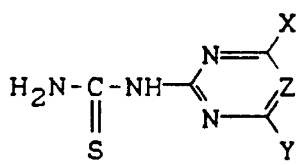

(II)

in welcher

X, Y und Z die oben angegebenen Bedeutungen haben,

mit Dialkylsulfaten der allgemeinen Formel (III)

$RO\text{-}SO_2\text{-}OR$ (III)

in welcher

R die oben angegebene Bedeutung hat,

gegebenenfalls in Gegenwart eines unpolaren Verdünnungsmittels bei Temperaturen zwischen 20 °C und 150 °C umsetzt

und die hierbei gebildeten Addukte der Formel (Ia)

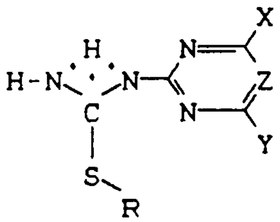 x $HO-SO_2-OR$ (Ia)

in welcher

R, X, Y und Z die oben angegebenen Bedeutungen haben,

nach Isolierung oder ohne Zwischenisolierung mit einem Säureakzeptor in Gegenwart eines aprotisch polaren Verdünnungsmittels beiTemperaturen zwischen 0 ° C und 50 ° C umsetzt.

Überraschenderweise gelingt es mit Hilfe des erfindungsgemäßen Verfahrens, die Verbindungen der Formel (I) auf relativ einfache Weise in hohen Ausbeuten herzustellen.

Das erfindungsgemäße Verfahren betrifft vorzugsweise die Herstellung von S-Alkyl-isothioureidoazinen der allgemeinen Formel (I), in welcher

R für Methyl oder Ethyl steht,

X und Y gleich oder verschieden sind und für Wasserstoff, Fluor, Chlor, Brom, für einen jeweils gegebenenfalls substituierten Rest der Reihe $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino oder Di-($C_1$-$C_2$-alkyl)-amino stehen, und

Z für Stickstoff oder eine CH-Gruppierung steht,

Insbesondere betrifft das erfindungsgemäße Verfahren die Herstellung von Verbindungen der Formel (I), in welcher

R für Methyl steht,

X für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht,

Y für Methyl, Trifluormethyl, Methoxy, Ethoxy oder Difluormethoxy steht, und

Z für Stickstoff oder eine CH-Gruppierung steht.

Verwendet man beispielsweise 4,6-Dimethyl-2-thioureidopyrimidin und Dimethylsulfat sowie in der Folgestufe Natriumhydroxid als Ausgangsstoffe, so kann der Reaktionsablauf durch das folgende Formelschema skizziert werden:

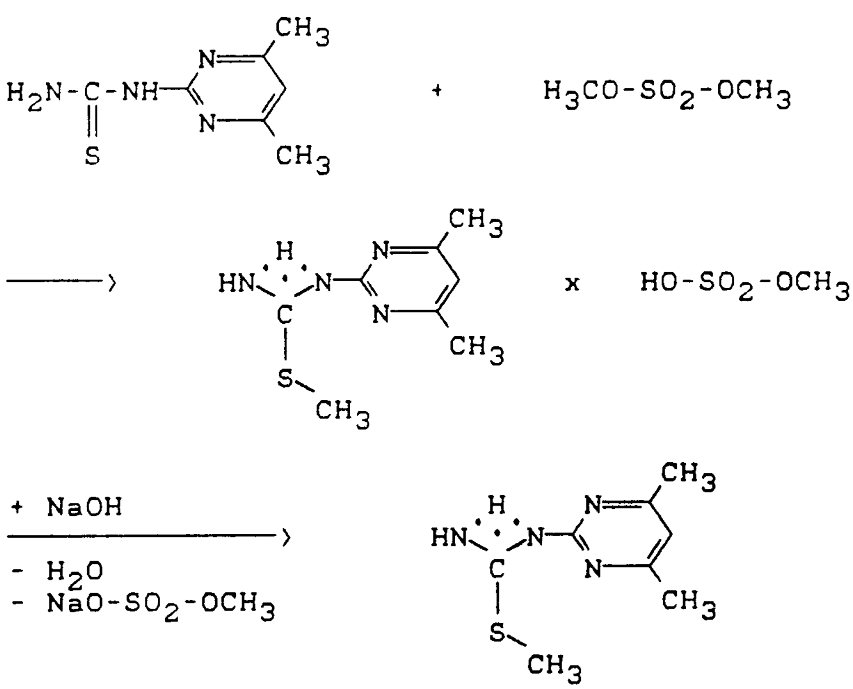

Die als Ausgangsstoffe zu verwendenden Thioureidoazine sind durch die Formel (II) allgemein definiert. In Formel (II) haben X, Y und Z vorzugsweise bzw. insbesondere diejenige Bedeutung, die bereits oben im Zusammenhang mit der Beschreibung der erfindungsgemäß herzustellenden Verbindungen der Formel (I) vorzugsweise bzw. als insbesondere bevorzugt für X, Y und Z angegeben wurden.

Die Ausgangsstoffe der Formel (II) sind bekannt und/oder können nach an sich bekannten Verfahren hergestellt werden (vgl. EP-A 173313).

Die weiter als Ausgangsstoffe benötigten Dialkylsulfate sind durch die Formel (III) allgemein definiert. In Formel (III) steht R vorzugsweise für Methyl oder Ethyl, insbesondere für Methyl.

Die Ausgangsstoffe der Formel (III) sind bekannte Synthesechemikalien.

Die erste Phase des erfindungsgemäßen Verfahrens wird gegebenenfalls in Gegenwart eines unpolaren Verdünnungsmittels durchgeführt. Als unpolare Verdünnungsmittel kommen vorzugsweise aromatische, insbesondere benzoide Kohlenwasserstoffe, welche gegebenenfalls 1 bis 3 Alkylsubstituenten mit jeweils 1 bis 3 Kohlenstoffatomen enthalten, in Betracht. Als Beispiele hierfür seien genannt: Benzol, Toluol, o-, m- und p-Xylol, Ethylbenzol, Propylbenzol, Cumol, 1,2,3-Trimethylbenzol, 1,2,4-Trimethylbenzol und 1,3,5-Trimethylbenzol. Toluol wird als Verdünnungsmittel ganz besonders bevorzugt.

Die Reaktionstemperaturen können in der ersten Phase des erfindungsgemäßen Verfahrens in einem größeren Bereich variiert werden. Im allgemeinen arbeitet man bei Temperaturen zwischen 20 °C und 150 °C, vorzugsweise bei Temperaturen zwischen 50 °C und 120 °C, insbesondere zwischen 70 °C und 110 °C.

Das erfindungsgemäße Verfahren wird im allgemeinen unter Normaldruck durchgeführt. Es ist jedoch auch möglich, unter erhöhtem oder vermindertem Druck zu arbeiten.

Als Säureakzeptoren können in der zweiten Phase des erfindungsgemäßen Verfahrens alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kaliumcarbonat oder -hydrogencarbonat sowie Calciumcarbonat, Alkalimetallacetate, wie Natrium-und Kalium-acetat, Alkalimetallalkoholate, wie Natrium-und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO). Kaliumcarbonat wird als Säureakzeptor ganz besonders bevorzugt.

Die Umsetzung der Addukte der Formel (Ia) mit einem Säureakzeptor als zweite Phase des erfindungsgemäßen Verfahrens wird in Gegenwart eines aprotisch polaren Verdünnungsmittels durchgeführt. Als solche Verdünnungsmittel kommen hierbei vorzugsweise Ketone, wie Aceton, Methylethylketon, Methylisopropylketon und Methylisobutylketon, Ester, wie Essigsäuremethylester und Essigsäureethylester, sowie Nitrile, wie Acetonitril und Propionitril in Betracht. Acetonitril wird als Verdünnungsmittel für die zweite Phase des erfindungsgemäßen Verfahrens ganz besonders bevorzugt.

Die Reaktionstemperaturen können in der zweiten Phase des erfindungsgemäßen Verfahrens in einem größeren Bereich variiert werden. Im allgemeinen arbeitet man bei Temperaturen zwischen 0 °C und 50 °C, vorzugsweise bei Temperaturen zwischen 10 °C und 40 °C, insbesondere zwischen 20 °C und 30 °C.

Zur Durchführung des erfindungsgemäßen Verfahrens setzt man auf 1 Mol Thioureidoazin der Formel (II) im allgemeinen zwischen 1,0 und 1,5 Mol, vorzugsweise zwischen 1,05 und 1,25 Mol, eines Dialkylsulfats der Formel (III) ein.

Die Ausgangsstoffe und gegebenenfalls das Verdünnungsmittel werden im allgemeinen bei Raumtemperatur vermischt und dann auf die erforderliche Reaktionstemperatur gebracht. Nach dem Ende der Umsetzung wird wieder auf Raumtemperatur (ca. 20 °C) abgekühlt. Im Falle der Verwendung eines Verdünnungsmittels kann das kristallin anfallende Addukt der Formel (Ia) durch Absaugen isoliert werden. Andernfalls wird das Produkt mit einem Verdünnungsmittel, wie z.B. tert-Butylmethylether und/oder Aceton, verrührt und abgesaugt. Bei lösungsmittelfreier Durchführung der ersten Phase des erfindungsgemäßen Verfahrens kann das Rohprodukt der Formel (Ia) auch ohne Zwischenisolierung weiter umgesetzt werden.

Zur Durchführung der zweiten Phase des erfindungsgemäßen Verfahrens wird das Addukt der Formel (Ia) mit einem Verdünnungsmittel und einem Säureakzeptor bei der erforderlichen Temperatur eine oder mehrere Stunden gerührt. Dann wird filtriert und vom Filtrat das Verdünnungsmittel unter vermindertem Druck sorgfältig abdestilliert. Man erhält das Produkt der Formel (I) als kristallinen Rückstand.

Die nach dem erfindungsgemäßen Verfahren herzustellenden S-Alkyl-isothioureidoazine der Formel (I) können als Zwischenprodukte zur Herstellung von Herbiziden verwendet werden (vgl. EP-A 121082 und EP-A 358018).

Herstellungsbeispiele:

Beispiel 1

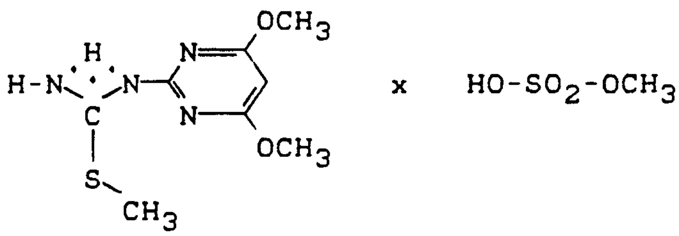

Eine Mischung aus 10,7 g (50 mMol) 4,6-Dimethoxy-2-thioureido-pyrimidin und 7,0 g (62 mMol) Dimethylsulfat wird 30 Minuten auf 80 °C bis 90 °C erhitzt. Dann wird auf 20 °C abgekühlt, das feste Produkt zerkleinert, mit tert-Butylmethylether verrührt und abgesaugt. Das Produkt wird mit Aceton verrührt und wieder abfiltriert.

Man erhält 15,6 g (92% der Theorie) 1:1-Addukt aus 4,6-Dimethoxy-2-(S-methyl-isothioureido)-pyrimidin und Schwefelsäure-monomethylester vom Schmelzpunkt 151 °C.

Beispiel 2

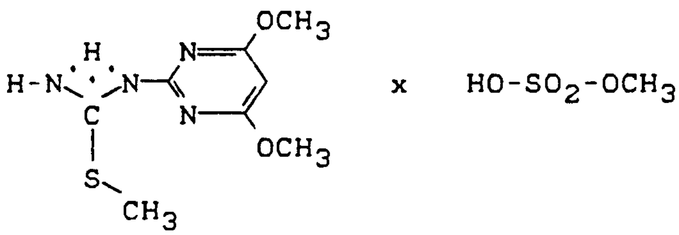

Eine Mischung aus 10,7 g (50 mMol) 4,6-Dimethoxy-2-thioureido-pyrimidin, 7,0 g (62 mMol) Dimethylsulfat und 100 ml Toluol wird 60 Minuten auf 100 °C erhitzt und dann auf Raumtemperatur (20 °C) abgekühlt. Das kristalline Produkt wird durch Absaugen isoliert.

Man erhält 15,7 g (92% der Theorie) 1:1-Addukt aus 4,6-Dimethoxy-2-(S-methyl-isothioureido)-pyrimidin und Schwefelsäure-monomethylester vom Schmelzpunkt 151 °C.

Beispiel 3

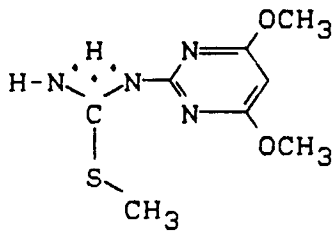

15 g (44 mMol) 1:1-Addukt aus 4,6-Dimethoxy-2-(S-methylisothioureido)-pyrimidin und Schwefelsäure-monomethylester (Herstellung vgl. Beispiele 1 und 2) werden in 100 ml Acetonitril mit 6,6 g (47 mMol) Kaliumcarbonat 60 Minuten bei 22 °C gerührt. Nach Filtration wird vom Filtrat das Lösungsmittel im Wasserstrahlvakuum sorgfältig abdestilliert,

Man erhält 9,6 g (96% der Theorie) 4,6-Dimethoxy-2-(S-methyl-isothioureido)-pyrimidin als kristallines Produkt vom Schmelzpunkt 126 °C.

Beispiel 4

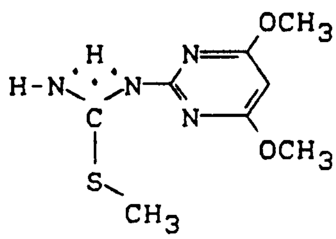

Eine Mischung aus 21,5 g (0,10 Mol) 4,6-Dimethoxy-2-thioureido-pyrimidin und 11,2 g (0,10 Mol) Dimethylsulfat wird 30 Minuten auf 90 °C bis 100 °C erhitzt. Nach Abkühlen auf 20 °C gibt man zum Produkt dieser Umsetzung 200 ml Acetonitril und rührt die Mischung 30 Minuten. Dann werden 15 g (0,11 Mol) Kaliumcarbonat dazu gegeben und die Mischung wird 4 Stunden bei 22 °C gerührt. Dann wird filtriert und vom Filtrat wird das Verdünnungsmittel im Wasserstrahlvakuum sorgfältig abdestilliert.

Man erhält 17,8 g (78% der Theorie) 4,6-Dimethoxy-2-(S-methyl-isothioureido)-pyrimidin als kristallines Produkt vom Schmelzpunkt 125 °C.

**Patentansprüche**

**1.** Verfahren zur Herstellung von S-Alkyl-isothioureidoazinen der allgemeinen Formel (I)

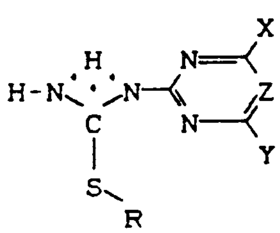

(I)

in welcher

R für Alkyl steht,

X und Y gleich oder verschieden sind und für Wasserstoff, Halogen oder für einen jeweils

gegebenenfalls substituierten Rest der Reihe Alkyl, Halogenalkyl, Alkoxyalkyl, Halogenalkoxy, Alkoxy, Alkylthio, Halogenalkylthio, Alkylamino oder Dialkylamino stehen, und

Z für Stickstoff oder eine CH-Gruppierung steht,

dadurch gekennzeichnet, daß man Thioureidoazine der allgemeinen Formel (II)

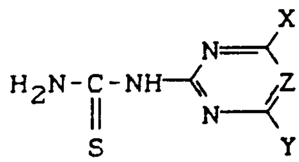

(II)

in welcher

X, Y und Z die oben angegebenen Bedeutungen haben,

mit Dialkylsulfaten der allgemeinen Formel (III)

$RO-SO_2-OR$ (III)

in welcher

R die oben angegebene Bedeutung hat,

gegebenenfalls in Gegenwart eines unpolaren Verdünnungsmittels bei Temperaturen zwischen 20°C und 150°C umsetzt

und die hierbei gebildeten Addukte der Formel (Ia)

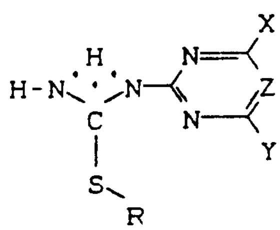

x $HO-SO_2-OR$ (Ia)

in welcher

R, X, Y und Z die oben angegebenen Bedeutungen haben,

nach Isolierung oder ohne Zwischenisolierung mit einem Säureakzeptor in Gegenwart eines aprotisch polaren Verdünnungsmittels bei Temperaturen zwischen 0°C und 50°C umsetzt.

2. Verfahren gemäß Anspruch 1 zur Herstellung von S-Alkyl-isothioureidoazinen der allgemeinen Formel (I), in welcher

R für Methyl oder Ethyl steht,

X und Y gleich oder verschieden sind und für Wasserstoff, Fluor, Chlor, Brom, für einen jeweils gegebenenfalls substituierten Rest der Reihe $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino oder Di-($C_1$-$C_2$-alkyl)-amino stehen, und

Z für Stickstoff oder eine CH-Gruppierung steht,

3. Verfahren gemäß Anspruch 1 zur Herstellung von S-Alkyl-isothioureidoazinen der allgemeinen Formel (I), in welcher

R für Methyl steht,

X für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht,

Y für Methyl, Trifluormethyl, Methoxy, Ethoxy oder Difluormethoxy steht, und

Z für Stickstoff oder eine CH-Gruppierung steht.

4. Verfahren gemäß Anspruch 1, dadurch gekennzeichnet, daß man die Umsetzung in der ersten Phase des Verfahrens bei Temperaturen zwischen 50°C und 120°C und in der zweiten Phase des Verfahrens

bei Temperaturen zwischen 10 ° C und 40 ° C durchführt.

5. Verfahren gemäß Anspruch 1, dadurch gekennzeichnet, daß man die Umsetzung in der ersten Phase des Verfahrens bei Temperaturen zwischen 70 ° C und 110 ° C und in der zweiten Phase des Verfahrens bei Temperaturen zwischen 20 ° C und 30 ° C durchführt.

6. Verfahren gemäß Anspruch 1, dadurch gekennzeichnet, daß man pro Mol Thioureidoazin der Formel (II) zwischen 1,0 und 1,5 Mol, vorzugsweise zwischen 1,05 und 1,25 Mol Dialkylsulfat der Formel (III) einsetzt.

7. Verfahren gemäß Anspruch 1, dadurch gekennzeichnet, daß man die erste Phase des Verfahrens in Gegenwart eines unpolaren Verdünnungsmittels, vorzugsweise von Toluol, durchführt.

8. Verfahren gemäß Anspruch 1, dadurch gekennzeichnet, daß man in der zweiten Phase des Verfahrens als aprotisch polares Verdünnungsmittel Acetonitril einsetzt.

9. Verfahren gemäß Anspruch 1, dadurch gekennzeichnet, daß man in der zweiten Phase des Verfahrens als Säureakzeptor Kaliumcarbonat einsetzt.

Europäisches Patentamt

# EUROPÄISCHER RECHERCHENBERICHT

Nummer der Anmeldung

EP 92 11 5271

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 358 018 (BAYER)<br>* das ganze Dokument *<br>----- | 1 | C07D239/52 |
| | | | **RECHERCHIERTE SACHGEBIETE (Int. Cl.5)** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1992 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument